# EUROPEAN PATENT APPLICATION

(11) **EP 4 261 543 A1**
(43) Date of publication of application: **18.10.2023**
(21) Application number: 21903166.3
(22) Date of filing: 22.11.2021
(51) Int. Cl.: G01N 35/00, G06Q 10/00

(54) **AUTOMATIC ANALYSIS SYSTEM, AND METHOD FOR INHERITING INFORMATION IN AUTOMATIC ANALYSIS SYSTEM**

(30) Priority: 09.12.2020 JP 2020204510
(71) Applicant: HITACHI HIGH-TECH CORPORATION, Tokyo 105-6409 (JP)
(72) Inventor: YOKOTSUKA, Satoshi, Tokyo 105-6409 (JP); AKUTSU, Masashi, Tokyo 105-6409 (JP); MISHIMA, Hiroyuki, Tokyo 105-6409 (JP)
(74) Representative: MERH-IP Matias Erny Reichl Hoffmann Patentanwälte PartG mbB
(86) International application number: PCT/JP2021/042755
(87) International publication number: WO 2022/124062

(57) **Abstract**

There is provided an automatic analysis system and an information takeover method in the automatic analysis system, in which repair and maintenance can be accurately executed at an appropriate timing even when a change occurs in a system configuration.

The automatic analysis system includes: two or more analyzers of a biochemical analyzer 110 and an immunoassay analyzer 210, which have unique identification information and cumulative information associated with the unique identification information, and analyze a sample; and a control device 300 that controls operations of the biochemical analyzers 110 and the immunoassay analyzer 210 and manages the unique identification information and the cumulative information of each of the biochemical analyzer 110 and the immunoassay analyzer 210, and when the biochemical analyzer 110 and the immunoassay analyzer 210 in a system are rearranged, the control device 300 takes over the cumulative information that the newly introduced biochemical analyzer 110 and immunoassay analyzer 210 have in a pre-rearrangement system based on the unique identification information, and manages cumulative information in a new system based on the taken-over cumulative information.

## Description

### Technical Field

The present invention relates to an automatic analysis system that performs a quantitative and qualitative analysis of a biological sample such as blood, plasma, serum, urine, and other body fluids (hereinafter, referred to as a sample or a specimen), and an information takeover method in such an automatic analysis system.

### Background Art

An example of a method for shortening time from a failure of a medium including a device database until a user can analyze again, by simplifying a recovery operation of the database before the failure without executing a registration operation or a calibration operation of a reagent residual amount, and checking integrity of the recovered database, PTL 1 describes arranging a backup of the database in an auxiliary operation unit and arranging a code for checking integrity of the database after recovery in an analysis measurement module.

### Citation List

### Patent Literature

PTL 1: JP2002-90369A

### Summary of Invention

### Technical Problem

An automatic analyzer that automatically performs a quantitative and qualitative analysis of a sample is used in many medical institutions or the like, particularly hospitals and clinical laboratory centers that need to process many patient samples in a short time.

As the automatic analyzer described above, various types of small, medium, and large automatic analyzers are developed in accordance with a sample processing capacity required by each medical institution.

As a software element that supports the automatic analyzer, there is software for operation unit and controller. An item requested by a user and a setting specified by the user with the operation unit are analyzed by the controller operating each mechanism in the automatic analyzer in accordance with the item and the setting.

In relation to a technique for acquiring cumulative information of such an automatic analyzer, PTL 1 describes constructing a backup of a database of device-specific information such as calibration curve information and reagent residual amount information using a storage medium external to a device, and restoring the database when the medium held by the device fails.

By acquiring cumulative information of an automatic analyzer, in particular, cumulative information such as a device adjustment value, device activation cumulative time, and lamp operation cumulative time, it is possible to determine the number of replaced components and necessity of repair and maintenance.

Further, as an automatic analyzer, there is an integrated device that improves a processing capability and the like by integrating a plurality of analyzers, with a group of equipment including one analysis instrument and equipment that executes a pre-analysis operation of the analysis instrument serving as a single device (hereinafter, referred to as an automatic analysis system).

In recent years, the analyzers connected to the automatic analysis system are not limited to the same type, but include various types. For example, there are a biochemical analyzer for measuring cholesterol and the like in blood, and an immunoassay analyzer for measuring infectious diseases and the like. There is also a device to which a plurality of different types or the same type of analyzers are connected. Accordingly, a trend is shifted from simply measuring large numbers of samples to measuring a wide variety of items.

Here, in recent years, the number of measurement items is increased due to combination of analyzers, and product life is longer due to improvement of durability of the analyzers. In such a situation, when a user replaces a device, there is a requirement of repairing and maintaining a recovered automatic analyzer again, renewing components, replacing consumable items, or the like.

However, the technique described in PTL 1 totally does not consider acquisition and takeover of cumulative information for maintenance when a device configuration in an automatic analysis system is changed, and is required to be improved.

For example, in a state in which in absence of cumulative information, operation time and the number of operations of the component are unknown in the repair and maintenance described above, and a visual check is performed for such a reason. However, in such a visual check, unnecessary components may be replaced, and a replacement component price is added to a product price. Similarly, there is a concern that a deteriorated component to be replaced may be left nonreplaced, resulting in a case of an initial failure.

Further, a user who owns a plurality of automatic analysis systems in which a plurality of analyzers are integrated requires to rearrange layout of a device configuration in accordance with an environment due to a change of layout in an examination room or the like. In such a case as well, it is necessary to continue accumulate cumulative information of each device after changing the layout of the device configuration, as in a case of resale. However, no such mechanism exists in the related art, and a technique for performing appropriate management is needed.

The invention provides an automatic analysis system and an information takeover method in the automatic analysis system, in which repair and maintenance can be accurately executed at an appropriate timing even when a change occurs in a system configuration.

### Solution to Problem

The invention includes a plurality of methods for solving the problems described above, one example of which is an automatic analysis system. The automatic analysis system includes: two or more analyzers having unique identification information and cumulative information associated with the unique identification information, and configured to analyze a sample; and a control device configured to control operations of the analyzers and manage the unique identification information and the cumulative information of each of the analyzers. When the analyzers in a system are rearranged, the control device takes over the cumulative information that a newly introduced analyzer has in a pre-rearrangement system based on the unique identification information, and manages cumulative information in a new system based on the taken-over cumulative information.

### Advantageous Effects of Invention

According to the invention, repair and maintenance can be accurately executed at an appropriate timing even when a change occurs in a system configuration. Problems, configurations, and effects other than those described above will be further clarified with the following description of embodiments.

### Brief Description of Drawings

[FIG. 1] FIG. 1 is a top view showing an outline of an overall configuration of an automatic analysis system according to Embodiment 1 of the present invention.
[FIG. 2] FIG. 2 is a front view of the automatic analysis system according to Embodiment 1.
[FIG. 3] FIG. 3 is a front view of an automatic analysis system of another aspect according to Embodiment 1.
[FIG. 4] FIG. 4 is a functional block diagram of the automatic analysis system according to Embodiment 1.
[FIG. 5] FIG. 5 is a diagram showing an example of an entire screen displayed by the automatic analysis system according to Embodiment 1.
[FIG. 6] FIG. 6 is a diagram illustrating an outline of a setup screen displayed in the automatic analysis system according to Embodiment 1.
[FIG. 7] FIG. 7 is a diagram illustrating an outline of a device configuration screen displayed in the automatic analysis system according to Embodiment 1.
[FIG. 8] FIG. 8 is a diagram illustrating an outline of another aspect of the device configuration screen displayed in the automatic analysis system according to Embodiment 1.
[FIG. 9] FIG. 9 is a diagram illustrating an outline of a system setting screen displayed in the automatic analysis system according to Embodiment 1.
[FIG. 10] FIG. 10 is a diagram illustrating an outline of a cumulative information setting screen displayed in the automatic analysis system according to Embodiment 1.
[FIG. 11] FIG. 11 is a flowchart of work when an analyzer is moved from a system 1 to a system 2 in the automatic analysis system according to Embodiment 1.
[FIG. 12] FIG. 12 is a flowchart when a user operates a cumulative information screen of the system 1 in a flow of FIG. 11.
[FIG. 13] FIG. 13 is a flowchart of a process of writing to a selected external medium in the flow of FIG. 11.
[FIG. 14] FIG. 14 is a flowchart when the user operates a cumulative information screen of the system 2 in the flow of FIG. 11.
[FIG. 15] FIG. 15 is a flowchart of a process of reading from the selected external medium in the flow of FIG. 11.

### Description of Embodiments

Hereinafter, embodiments of an automatic analysis system and an information takeover method in the automatic analysis system according to the invention will be described with reference to the drawings. In the drawings used in the present specification, the same or corresponding components are denoted by the same or similar reference numerals, and repeated description of these components may be omitted.

### <Embodiment 1>

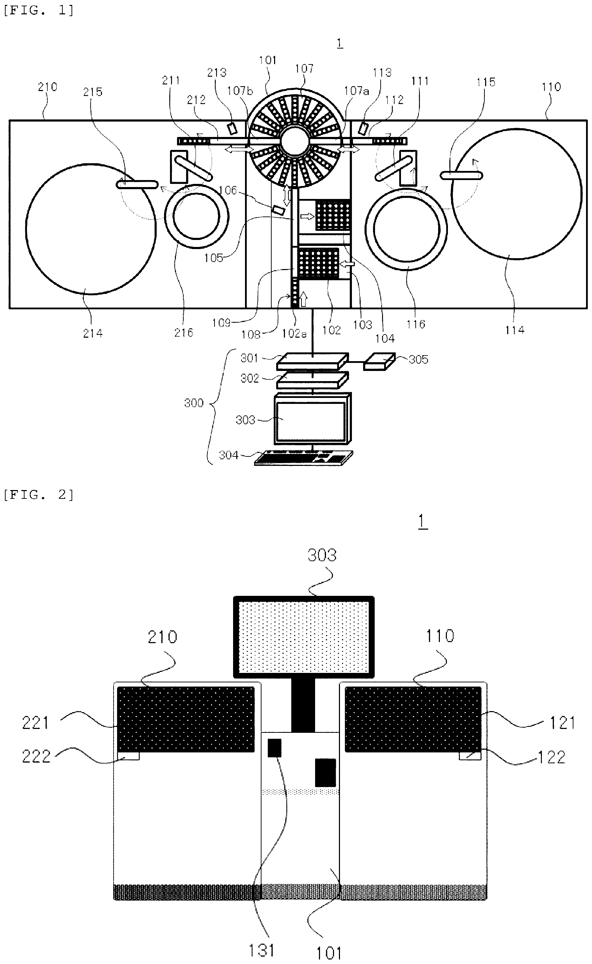

An automatic analysis system and an information takeover method in the automatic analysis system according to Embodiment 1 of the invention will be described with reference to FIG. 1 to FIG. 15.

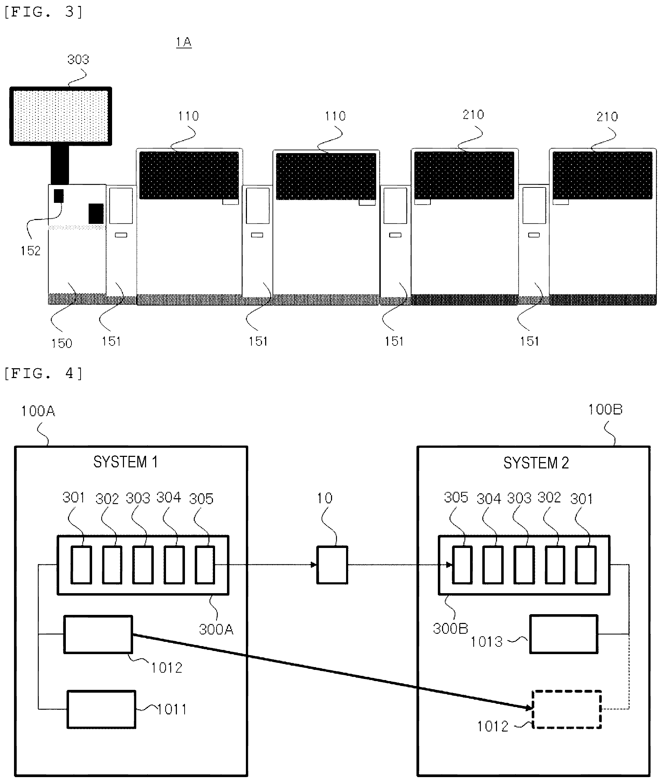

First, an overall configuration of the automatic analysis system according to the present embodiment will be described with reference to FIG. 1 to FIG. 3. FIG. 1 and FIG. 2 are views showing the overall configuration of the automatic analysis system according to the present embodiment, in which FIG. 1 shows an outline when viewed from top and FIG. 2 shows an outline when viewed from front. FIG. 3 is a view showing an overall configuration of an automatic analysis system to which three or more analyzers according to the present embodiment are connected, and shows an outline when viewed from front.

An automatic analysis system 1 according to the present embodiment shown as an example in FIG. 1 schematically includes a plurality of biochemical analyzer 110 and immunoassay analyzer 210 (two in FIG. 1), a sampler device 101 that conveys a sample rack on which one or more sample containers containing samples to be analyzed by the biochemical analyzer 110 and the immunoassay analyzer 210 are mounted, and a control device 300 that controls entire operations of the automatic analysis system 1.

Here, the sample rack is mounted with one or more sample containers containing samples to be subjected to qualitative and quantitative analysis in the biochemical analyzer 110 and the immunoassay analyzer 210.

The sample rack includes at least a sample rack on which a sample container containing a sample (a normal sample) to be analyzed with a normal priority is mounted (hereinafter, simply referred to as a sample rack 102), and a sample rack on which a sample container containing an emergency sample having a higher emergency of analysis measurement than the sample rack 102 is mounted (hereinafter referred to as an emergency sample rack 102a when particularly distinguishing from the sample rack 102).

The sampler device 101 in FIG. 1 is a device that conveys the sample rack 102 loaded into the automatic analysis system 1 between the biochemical analyzer 110 and the immunoassay analyzer 210. The sampler device 101 includes a sample rack supply unit 103, an emergency sample rack loading unit 108, a transport line 105, an emergency sample rack waiting area 109, a sample identification device 106, a rack rotor 107, a sample rack accommodating unit 104, and the like.

The transport line 105 is, for example, a belt conveyor transport mechanism that reciprocally transports the sample rack 102 and the emergency sample rack 102a.

The emergency sample rack loading unit 108 is provided adjacent to the transport line 105, and is a region for loading the emergency sample rack 102a.

The sample rack supply unit 103 is provided adjacent to the transport line 105 on one end side of the transport line 105 with respect to the emergency sample rack loading unit 108, and is a region for supplying the sample rack 102 of the normal sample.

The sample rack accommodating unit 104 is provided adjacent to the transport line 105 on one end side of the transport line 105 with respect to the sample rack supply unit 103, and is a region for accommodating the sample rack 102.

The emergency sample rack waiting area 109 is provided on the transport line 105 on the other end side of the transport line 105 with respect to the sample rack accommodating unit 104, and is a region for temporarily waiting the emergency sample rack 102a.

The sample identification device 106 is a mechanism that reads and identifies an identification medium (not shown) such as an RFID or a barcode provided in the sample rack 102 transported on the transport line 105 and the sample container in order to query analysis request information related to the sample contained in the sample container mounted on the sample rack 102.

The rack rotor 107 is disposed at one end of the transport line 105. The rack rotor 107 includes one or more slots 107a and 107b on which the sample rack 102 and the like can be mounted. The rack rotor 107 is a mechanism that transmits and receives the sample rack 102 and the like to and from one end of the transport line 105 and one end of each of dispensing lines 112, 212 of the biochemical analyzer 110 and the immunoassay analyzer 210.

For example, the rack rotor 107 is structured to rotate in clockwise and counterclockwise directions, and a rotation operation of the rack rotor 107 is appropriately controlled such that processing is started in an order in which the sample racks 102 are loaded, or such that processing can be started before a sample rack 102 loaded earlier when a sample rack 102 with high priority is loaded.

The biochemical analyzer 110 and the immunoassay analyzer 210 are units that perform a qualitative and quantitative analysis by sampling (dispensing) a sample contained in a sample container mounted on a sample rack 102. The biochemical analyzer 110 and the immunoassay analyzer 210 include the dispensing lines 112, 212, sample identification devices 113, 213, reaction disks 116, 216, sample dispensing mechanisms 111, 211, reagent disks 114, 214, reagent dispensing mechanisms 115, 215, a measurement unit (not shown), and the like, respectively.

The dispensing lines 112 and 212 adopt a reciprocating transport mechanism that draws the sample rack 102 from the sampler device 101 to the biochemical analyzer 110 and the immunoassay analyzer 210 and delivers the sample rack 102 from the biochemical analyzer 110 and the immunoassay analyzer 210 to the sampler device 101. For example, the dispensing lines 112 and 212 adopt a belt conveyor mechanism.

The dispensing lines 112 and 212 are exemplified as a case in which a belt conveyor transport mechanism is adopted, but may adopt a configuration of performing transportation with a projection structure driven along the dispensing lines 112 and 212 being fitted into a recess provided in advance in the sample rack 102. This configuration also applies to the transport line 105.

The sample identification devices 113, 213 are provided adjacent to the other end side of the dispensing lines 112, 212. The sample identification devices 113, 213 are mechanisms that read and identify an identification medium (not shown) such as an RFID or a barcode provided in the sample rack 102 transported into the dispensing lines 112, 212 and the sample container in order to match analysis request information for a sample accommodated in the sample rack 102.

The sample dispensing mechanisms 111, 211 are mechanisms that dispense a sample from a sample container of the sample rack 102 transported to dispensing positions on the dispensing lines 112, 212 into reaction containers of the reaction disks 116, 216.

The reagent dispensing mechanisms 115, 215 are mechanisms that dispense a reagent contained in reagent containers of the reagent disks 114, 214 into reaction containers of the reaction disks 116, 216.

The measurement unit is a mechanism that measures a mixed liquid (a reaction liquid) of a sample and a reagent dispensed into a reaction container and performs a qualitative and quantitative analysis.

The present embodiment assumes that the biochemical analyzer 110 is a device for a biochemical test, and the immunoassay analyzer 210 is a device for an immunological test, but is not limited to such a case, and may use devices for the same test item.

Further, the present embodiment has unique identification information represented by a device serial number or the like and cumulative information associated with the unique identification information defined for each of the biochemical analyzer 110 and the immunoassay analyzer 210. The unique identification information and the cumulative information are provided per unit (the biochemical analyzer 110 and the immunoassay analyzer 210). Details of the unique identification information and the cumulative information will be described later.

In addition, a measurement unit for electrolyte concentration measurement may be provided in the biochemical analyzer 110, or a measurement unit for blood coagulation analysis or the like may be appropriately provided in each device according to a specification environment.

Further, as shown in FIG. 3, when purposes (test items) are the same, a plurality of analyzers may be the same analyzers, and processing capacities may be maintained to be the same. Further, when only purposes are different, a plurality of different analyzers may be connected.

The control device 300 is a device that controls the entire operations of the automatic analysis system 1, including the devices including the biochemical analyzer 110, the immunoassay analyzer 210, and the sampler device 101, and is a computer including a CPU, a memory, and the like.

The control device 300 includes a display unit 303, an input unit 304, a storage unit 302, a controller 301, an output unit 305, and the like.

The display unit 303 is a display device such as a liquid crystal display that displays various kinds of information such as an input screen of various parameters and settings, analysis test data of an initial test or a re-test, a measurement result, and reagent information in the automatic analysis system 1, and displays various kinds of information such as information related to maintenance of the biochemical analyzer 110, the immunoassay analyzer 210, and the sampler device 101. The display unit 303 may be a touch panel display unit that also serves as an input unit 304 described later.

The input unit 304 includes a keyboard and a mouse for inputting various data such as various parameters and settings, analysis request information, and instructions to start analysis or the like.

The storage unit 302 is a record medium such as a semiconductor memory such as a flash memory or a magnetic disk such as an HDD that records a measurement result of a sample loaded into the automatic analysis system 1, analysis request information on a sample contained in a sample container mounted on each sample rack, and the like. The storage unit 302 also records various parameters and set values for controlling operations of each device in the automatic analysis system 1, various computer programs for executing various processing and the like to be described later, and the unique identification information and cumulative information of each of the biochemical analyzer 110 and the immunoassay analyzer 210.

The controller 301 is a part that controls entire operations of the control device 300 and the automatic analysis system 1 which includes the biochemical analyzer 110, the immunoassay analyzer 210, and the sampler device 101. The controller 301 is the CPU described above or the like.

The output unit 305 includes a connector that connects an external medium 10 (see FIG. 4) and an output terminal such as a LAN port that connects to an external system outside the automatic analysis system 1 wirelessly or by wire.

The above is the configuration of the automatic analysis system 1.

A sample analysis processing of a sample performed by the automatic analysis system 1 as described above is generally executed in the following procedure.

A user gives an analysis instruction to the automatic analysis system 1 using the display unit 303 and the input unit 304. The analysis instruction is stored in the storage unit 302 and transmitted to a target analyzer among the sampler device 101, the biochemical analyzer 110, and the immunoassay analyzer 210 via the control device 300. The target device performs analysis operations as follows in accordance with the received analysis instruction.

The sampler device 101 sends out the sample racks 102 disposed on the sample rack supply unit 103 one rack at a time onto the transport line 105, and transports the sample rack 102 into the rack rotor 107.

The sample rack 102 transported to the rack rotor 107 is transported to the dispensing line 112 of the biochemical analyzer 110 or the dispensing line 212 of the immunoassay analyzer 210 in accordance with a measurement item requested by the control device 300.

When the sample rack 102 arrives at the dispensing lines 112 and 212, a dispensing operation is performed on each sample mounted on the sample rack 102 by the sample dispensing mechanisms 111 and 211.

When the measurement item is a biochemical item, the sample dispensing mechanism 111 ejects an aspirated sample to a reaction container on the reaction disk 116. Thereafter, a reagent aspirated from the reagent disk 114 by the reagent dispensing mechanism 115 is further added to the reaction container and stirred. Thereafter, absorbance or the like are measured by the measurement unit, and a measurement result is transmitted to the controller 301 of the control device 300.

The reaction container used for the analysis is cleaned with water, an alkaline detergent, and an acidic detergent dispensed from a cleaning mechanism (not shown) and used for a next analysis.

Further, when the measurement item is an immunological item, the reagent dispensed from the reagent disk 214 by the reagent dispensing mechanism 215 is ejected to a reaction container on the reaction disk 216, and a sample is further added to the reaction container by the sample dispensing mechanism 211 and stirred. Thereafter, after processing such as magnetic separation is performed as necessary, measurement is performed by the measurement unit, and a measurement result is transmitted to the controller 301 of the control device 300.

The controller 301 performs processing such as arithmetic processing on the transmitted measurement result to obtain a concentration of a specific component in the sample, and displaying a result on the display unit 303 or the like or storing the result in the storage unit 302.

Further, as shown in FIG. 2, a display unit 303 is provided above the sampler device 101. The sampler device 101 includes a barcode reader 131, which is a device for reading a barcode.

Further, a top cover 121 is provided on top of the biochemical analyzer 110, and a top cover 221 is provided on top of the immunoassay analyzer 210. The top covers 121 and 221 can be opened and closed, and FIG. 1 is a top view of an opened state. Further, opening and closing detectors 122, 222 that detect opening of the top covers 121, 221 are provided in the analyzers.

The automatic analysis system 1 is not limited to the configuration shown in FIG. 1. As shown in FIG. 3, an automatic analysis system 100A may include a plurality of biochemical analyzers 110 and immunoassay analyzers 210.

The automatic analysis system 100A shown in FIG. 3 includes two biochemical analyzers 110, two immunoassay analyzers 210, one sampler device 150, four rack accommodating units 151, and the control device 300 that controls the entire operations of the automatic analysis system 1.

In FIG. 3, the sampler device 150 includes a barcode reader 152 which is a device for reading a barcode.

Next, a characteristic control of the automatic analysis system 1 according to the present embodiment will be described in detail with reference to FIG. 4 and subsequent figures.

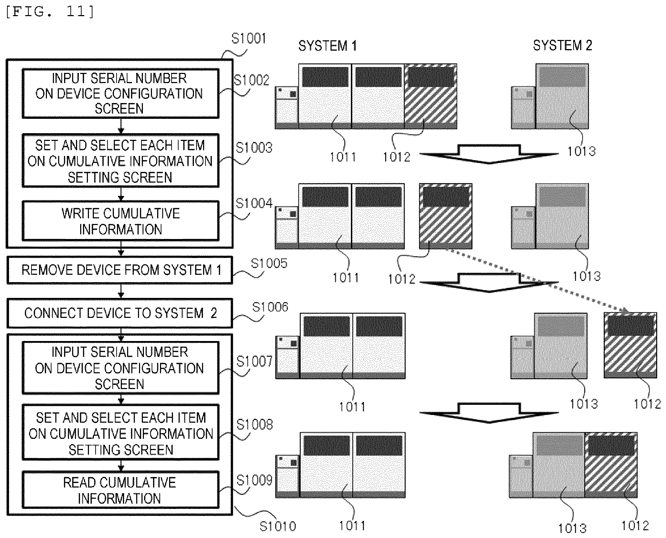

First, a functional block diagram related to the characteristic control of the automatic analysis system 1 will be described with reference to FIG. 4. FIG. 4 is a functional block diagram of an automatic analysis system. FIG. 4 illustrates a case in which an analysis unit 1012 is relocated from an automatic analysis system 100A of a system 1 to an automatic analysis system 100B of a system 2 as in FIG. 11 described later.

As shown in FIG. 4, in the automatic analysis system 100A of the system 1 including an analysis unit 1011 and the analysis unit 1012 to be relocated, a controller 301, a storage unit 302, a display unit 303, an input unit 304, and an output unit 305 of a control device 300A are related to each other. In the automatic analysis system 100A of the system 2 including the analysis unit 1013 and serving as a relocation destination of the analysis unit 1012, a controller 301, a storage unit 302, a display unit 303, an input unit 304, and an output unit 305 of a control device 300B are related to each other. The external medium 10 can be connected to each of the output unit 305 of the automatic analysis system 100A and the output unit 305 of the automatic analysis system 100B.

Examples of the external medium 10 include a USB medium, a CD medium, a DVD medium, an external HHD, and an external SSD, and the like, and any one or more of them can be used.

In such a configuration, when the analysis unit 1012 to be relocated, which is either the biochemical analyzer 110 or the immunoassay analyzer 210 in the system, is rearranged from the automatic analysis system 100A to the automatic analysis system 100B, the control device 300 according to the present embodiment performs control to take over cumulative information in the automatic analysis system 100A before a rearrangement in the analysis unit 1012 newly introduced in the automatic analysis system 100B based on unique identification information, and manage cumulative information in the new automatic analysis system 100B based on the taken-over cumulative information.

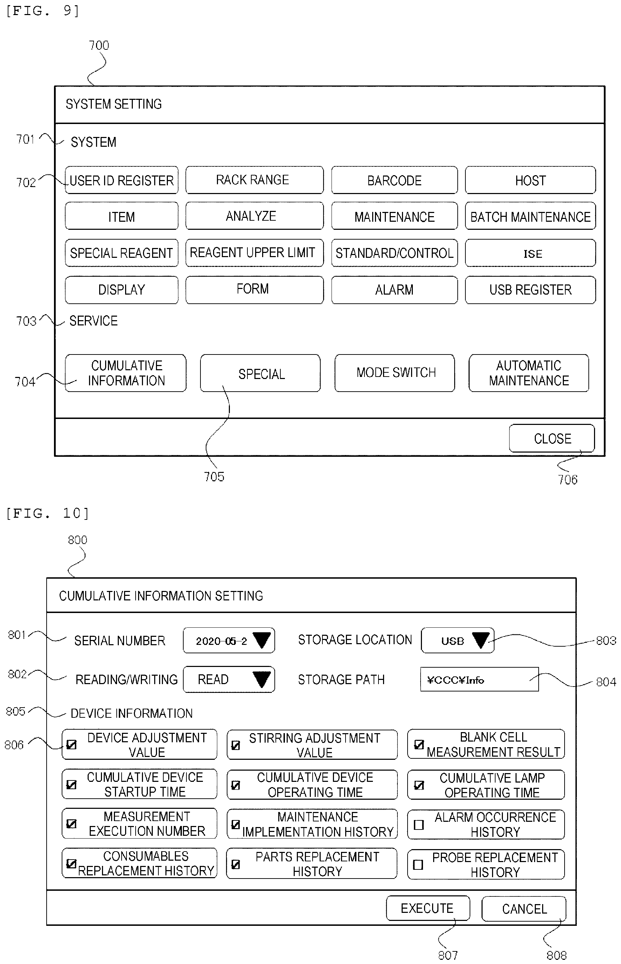

Here, the "cumulative information" in the invention is, for example, information such as an adjustment value, a cell blank measurement result, component replacement/maintenance information, an alarm generation record, and an operation time, and is information displayed in respective cumulative information selection checkboxes 806 of a cumulative information setting screen 800 in FIG. 10 described later.

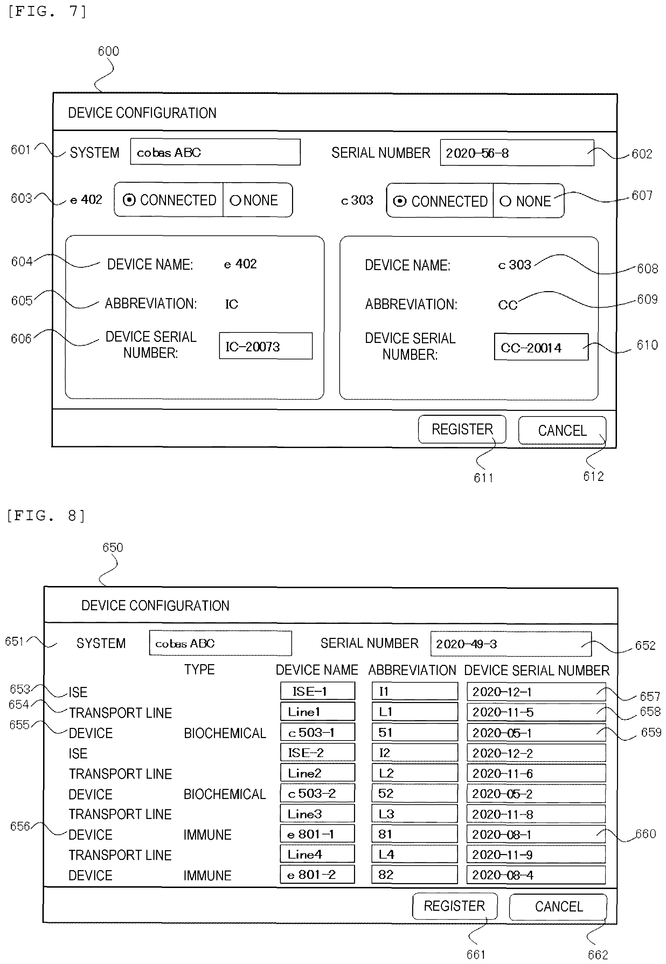

Further, the control device 300 causes the display unit 303 to display device configuration screens 600, 650 (see FIG. 7, FIG. 8, respectively) for setting unique identification information, and a cumulative information setting screen 800 (see FIG. 10) on which cumulative information can be read and written.

Further, the control device 300 can write and read unique identification information and cumulative information to and from the external medium 10. The unique identification information and the cumulative information can be written and read for each analyzer, and for this purpose, it is desirable that serial numbers of the system and the analyzer be assigned numbers in such a way that the serial numbers are uniquely determined. Accordingly, the unique identification information and the cumulative information including the serial numbers or the like of the system and the analyzer can be prevented from overlapping with information of the same device when crossing systems, and the cumulative information can be taken-over more stably.

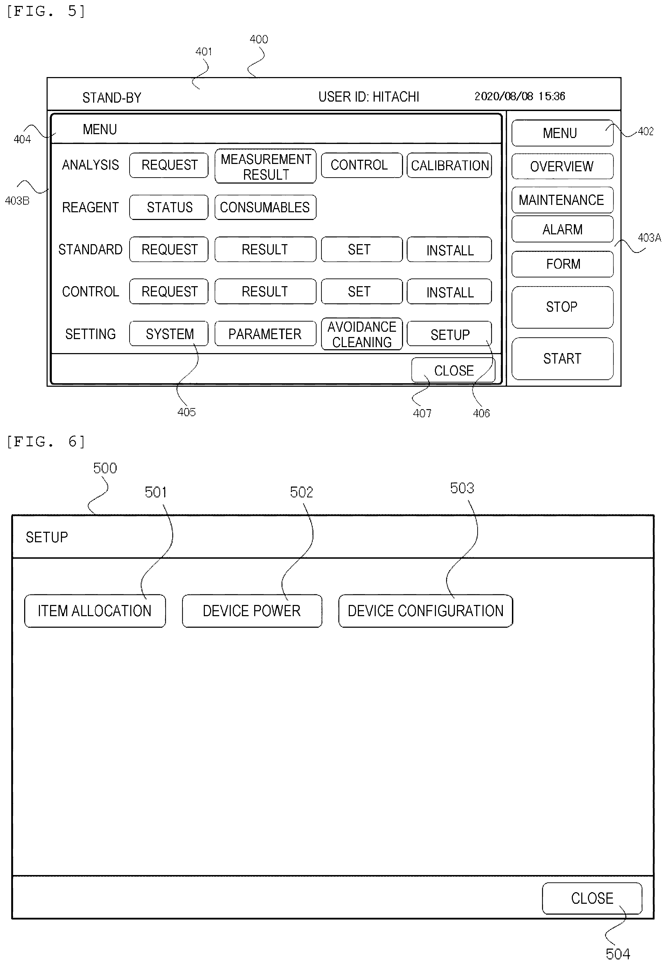

Next, a display control of a screen displayed on the display unit 303 will be described in detail with reference to FIG. 5 and subsequent figures. First, details of an entire screen 400, the device configuration screen 600, the device configuration screen 650, and the cumulative information setting screen 800 of the automatic analysis systems 1 and 1A displayed on the display unit 303 will be described with reference to FIG. 5 to FIG. 10.

FIG. 5 is the entire screen 400 displayed on the display unit 303 of the automatic analysis system 1. The entire screen 400 shown in FIG. 5 includes a status region 401 in which a system state, an ID of a user of user currently using the system, current time, and the like are displayed, a global region 403A in which buttons capable of displaying respective operation screens are disposed, and a local region 403B in which a screen selected in the global region 403A is displayed.

On the entire screen 400, a screen corresponding to a button selected in the global region 403A is displayed in the local region 403B, and in an example of FIG. 5, a menu button 402 is selected and a menu screen 404 is displayed. Further, when an overview button is selected, an overview screen (not shown) is displayed. When a maintenance button is selected, a maintenance screen (not shown) is displayed. When an alarm button is selected, an alarm screen (not shown) is displayed. When a form button is selected, a form screen (not shown) is displayed. When a stop button is selected, a stop selection screen (not shown) is displayed. When a start button is selected, a start screen (not shown) is displayed.

A plurality of buttons for displaying various screens for performing various operations of the automatic analysis system 1 are disposed on the menu screen 404. An analysis region includes a request button, a measurement result button, a control button, and a calibration button, and each screen is displayed in response to selection of a corresponding one of the buttons. A reagent region includes a status button and a consumable button. A standard region includes a request button, a result button, a setting button, and an install button. A control region includes a request button, a result button, a setting button, and an install button. A setting region includes a system button, a parameter button, an avoidance cleaning button, and a setup button. Further, when a close button 407 is selected, the menu screen 404 is closed.

Further, on the entire screen 400, when a system button 405 is selected, a system setting screen 700 shown in FIG. 9 is displayed, and when a setup button 406 is selected, a setup screen 500 shown in FIG. 6 is displayed.

FIG. 6 is a diagram showing an example of a setup screen. When the setup button 406 on the menu screen 404 of the entire screen 400 shown in FIG. 5 is selected, the setup screen 500 shown in FIG. 6 is displayed.

As shown in FIG. 6, an item allocation button 501, a device power button 502, a device configuration button 503, and a close button 504 are displayed on the setup screen 500. When the item allocation button 501 is selected, an item allocation screen (not shown) is displayed, and it is possible to allocate which analysis item is measured by which analyzer. When the device power button 502 is selected, a device power screen (not shown) is displayed, and power of each device can be switched between ON and OFF. When the close button 504 is selected, the setup screen 500 is closed, and the screen returns to the menu screen 404.

When the device configuration button 503 is selected, in a case in which the device configuration is a device configuration of the automatic analysis system 1 as shown in FIG. 2, the device configuration screen 600 as shown in FIG. 7 is displayed. Further, when the device configuration is the automatic analysis system 100A as shown in FIG. 3, the device configuration screen 650 as shown in FIG. 8 is displayed. This screen layout is suitable for each product.

The device configuration screen 600 shown in FIG. 7 is a common screen layout in a case of a configuration layout in the automatic analysis system 1 as shown in FIG. 2. A system name field 601 is a region for inputting a system name of the automatic analysis system 1, and a system serial number field 602 is a region for inputting a system serial number.

When the immunoassay analyzer 210 is connected to a system configuration, an immune device connection radio button 603 is selected as "connected", and when the immunoassay analyzer 210 is not connected, the immune device connection radio button 603 is selected as "none". Similarly, when the biochemical analyzer 110 is connected to the system configuration, a biochemical device connection radio button 607 is selected as "connected", and when the biochemical analyzer 110 is not connected, the biochemical device connection radio button 607 is selected as "none".

A device serial number to be input on the device configuration screen 600 and the device configuration screen 650 described later may be input by a service representative using the input unit 304. Further, there is also a method of enabling the unique identification information to be written out as a barcode in the control device 300, converting the device serial number into a barcode, managing the unique identification information using the barcode readers 131 and 152, and storing the unique identification information in the corresponding device serial number.

As for these device serial numbers or the like, it is desirable for a manufacturer of a system to assign a number for each device in advance that does not overlap with those of other systems and devices, and to input the number in such a way that the number do not overlap with those of other systems and devices.

When the immunoassay analyzer 210 is connected in FIG. 7, an immune device name 604 and an immune device abbreviation 605 are displayed, and an immune device serial number 606 can be set. Further, when the biochemical analyzer 110 is connected, a biochemical device name 608 and a biochemical device abbreviation 609 are displayed, and a biochemical device serial number 610 can be set. When input and setting are completed, a registration button 611 is selected, and when the input and setting are to be discarded, a cancel button 612 is selected.

The device configuration screen 650 shown in FIG. 8 is a common screen layout in a case of a configuration layout in the automatic analysis system 100A as shown in FIG. 3. A system name field 651 is a region for inputting a system name of the automatic analysis system 100A, and a system serial number field 652 is a region for inputting a system serial number.

An ISE device information region 653 includes a device name input field and a device abbreviation input field. An ISE device serial number 657 is for inputting a device serial number. Further, similarly, a sampler device information region 654 includes a device name input field and a device abbreviation input field. A sampler device serial number 658 is for inputting a device serial number. A biochemical device information region 655 includes a device name input field and a device abbreviation input field. A biochemical device serial number 659 is for inputting a device serial number. An immune device information region 656 includes a device name input field and a device abbreviation input field. An immune device serial number 660 is for inputting a device serial number. When input and setting are completed, a registration button 661 is selected, or a cancel button 662 is selected for discarding the input and setting and closing the screen.

FIG. 9 shows the system setting screen 700, which is displayed when the system button 405 on the menu screen 404 is selected. As shown in FIG. 9, a system setting button region 701 and a service setting button region 703 are displayed on the system setting screen 700.

The system setting button region 701 is provided with screen display buttons 702 for respective system settings for displaying screens on which various settings of the system can be changed.

The screen display buttons 702 for respective system settings include a user ID registration button, a rack range button, a barcode button, a host button, an item button, an analysis button, a maintenance button, a collective maintenance button, a special reagent button, a reagent upper limit button, a standard/control button, an ISE button, a display button, a form button, an alarm button, a USB registration button, and the like.

The service setting button region 703 is provided with screen display buttons 705 for respective service settings for displaying screens at a service level that allows setting by the representative. The screen display buttons 705 for respective service settings are each provided with a cumulative information button 704, a special button, a mode switch button, an automatic maintenance button, and the like. When a close button 706 is selected, the screen returns to the menu screen 404 shown in FIG. 5.

The cumulative information setting screen 800 shown in FIG. 10 is a screen for writing and reading cumulative information of a device, and is displayed by selecting the cumulative information button 704 on the system setting screen 700 shown in FIG. 9.

When a serial number of a target device is selected in a device serial number selection field 801, for the selected device, it is possible to select, in an information reading and writing selection field 802, whether cumulative information is to be written from the device to the external medium 10 or the like or to be read from the external medium 10 or the like to the device.

An information storage location selection field 803 is a region for selecting where to perform reading and writing of the cumulative information selected in the information reading and writing selection field 802. An information storage path input box 804 is a region for inputting a path for storing in the external medium 10 selected in the information storage location selection field 803.

A device cumulative information region 805 is a region for selecting on/off of a check for the respective cumulative information selection checkboxes 806. In a case in which the check is on, cumulative information is set to be read and written, and in a case in which the check is off, cumulative information is set not to be read or written.

When settings of the device serial number selection field 801, the information reading and writing selection field 802, the information storage location selection field 803, the information storage path input box 804, and the respective cumulative information selection checkboxes 806 are completed on the cumulative information setting screen 800, an execute button 807 is selected. When reading or writing is not to be performed, a cancel button 808 is selected.

Next, a specific example of takeover processing will be described with reference to FIG. 11 to FIG. 15. FIG. 11 is a flowchart showing a flow when the relocated analysis unit 1012 is moved from the system 1 to the system 2. A system execution entity for respective flowcharts in FIG. 11 and subsequent figures is the controller 301 of the control device 300.

As shown in FIG. 11, first, a screen operation of the system 1 displayed on the display unit 303 is performed by the input unit 304 of the control device 300A of the automatic analysis system 100A of the system 1 (step S1001).

In step S1001, first, the system serial number field 652 and the device serial numbers such as the ISE device serial number 657, the sampler device serial number 658, the biochemical device serial number 659, the immune device serial number 660, and the like, which are the device serial numbers of the respective analyzers, are input on the device configuration screen 650 (step S1002).

Then, each item is set and selected on the cumulative information setting screen 800 (step S1003), and set cumulative information is written (step S1004). In a case of step S1002, as for the device configuration screen 600, the system serial number field 602, the immune device serial number 606 and the biochemical device serial number 610, which are the device serial numbers of the respective analyzers, are input.

Next, the analysis unit 1012 is removed from the automatic analysis system 100A of the system 1 including the analysis units 1011 and 1012 and the control device 300A (step S1005), and the removed analysis unit 1012 is connected to the automatic analysis system 100B of the system 2 including the analysis unit 1013 and the control device 300B (step S1006).

After completion of step S1006, a screen operation of the system 2 displayed on the display unit 303 is performed by the input unit 304 of the control device 300B of the automatic analysis system 100B of the system 2 (step S1010).

In step S1010, first, the system serial number field 652, the ISE device serial number 657, the sampler device serial number 658, the biochemical device serial number 659, and the immune device serial number 660, which are the device serial numbers of the respective analyzers, on the device configuration screen 650 are input (step S1007). In step S1007, in a case of the device configuration screen 600, the system serial number field 602, the immune device serial number 606 and the biochemical device serial number 610, which are the device serial numbers of the respective analyzers, are input.

Next, each item is set and selected on the cumulative information setting screen 800 (step S1008), and when information input in step S1007 matches cumulative information set in step S1008, the cumulative information is read (step S1009), and settings of the system 2 are completed.

After reading is completed, for the cumulative information of the analysis unit 1012, the automatic analysis system 100B of the system 2 manages cumulative information based on the cumulative information taken over from the automatic analysis system 100A of the system 1 in the procedure described above.

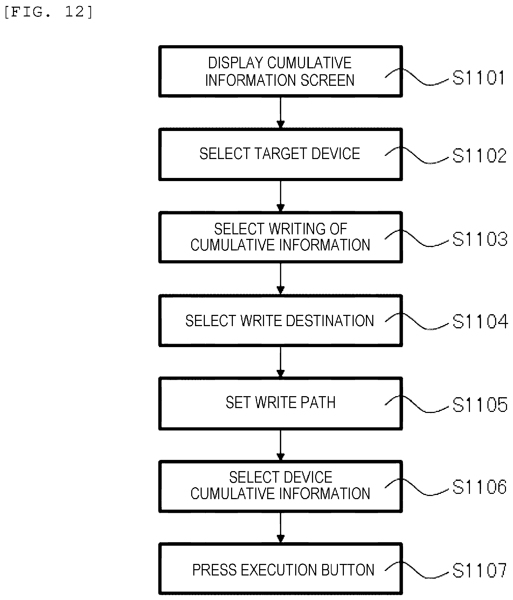

FIG. 12 is a flowchart of operating settings on the cumulative information setting screen 800 in step S1003 in FIG. 11.

First, a cumulative information button is selected on the system setting screen 700, and the cumulative information setting screen 800 is displayed (step S1101).

Next, the analyzer of the analysis units 1011 and 1012 of system 1 from which the cumulative information is to be acquired is selected in the device serial number selection field 801 (step S1102). Then, in order to acquire cumulative information from the device, writing is selected in the information reading and writing selection field 802 (step S1103), and the external medium 10 the cumulative information in which is to be stored is selected in the information storage location selection field 803 (step S1104).

Then, in order to determine an output path of the external medium 10 specified for the cumulative information, a path is input in the information storage path input box 804 (step S1105), and cumulative information to be written to the external medium 10 is checked from the respective cumulative information selection checkboxes 806 (step S1106).

Finally, when the execute button 807 is pressed, operations are completed on the cumulative information setting screen 800, and a process of writing to the external medium 10 is started (step S1107).

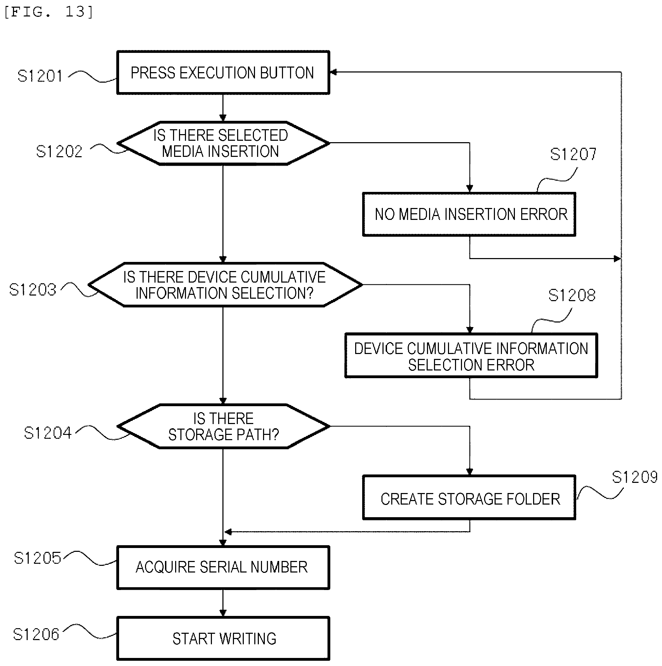

FIG. 13 shows an internal processing flow executed after step S1107 in FIG. 12.

In FIG. 13, first, when it is detected that the execute button 807 is selected on the cumulative information setting screen 800 (step S1201), in order to write cumulative information to the external medium 10 selected in the information storage location selection field 803, it is checked whether the external medium 10 is inserted into the output unit 305 (step S1202).

When the external medium 10 is not inserted, an error screen (not shown) is displayed (step S1207), and the processing returns to step S1201. On the other hand, when the external medium 10 is inserted, the processing proceeds to step S1203.

Then, it is checked whether a write target is checked in the respective cumulative information selection checkboxes 806 (step S1203). When none are checked, an error screen is displayed (step S1208), and the processing returns to step S1201. On the other hand, when a write target is checked, the processing proceeds to step S1204.

Next, when the cumulative information is written to the external medium 10, it is checked whether the path input in the information storage path input box 804 exists (step S1204). When the folder of the path does not exist, a folder designated by the path is created (step S1209), and the folder of the path exists, the processing proceeds to step S1205.

Then, a serial number selected in the device serial number 801 is acquired as key information (step S1205), and writing is executed to the external medium 10 (step S1206).

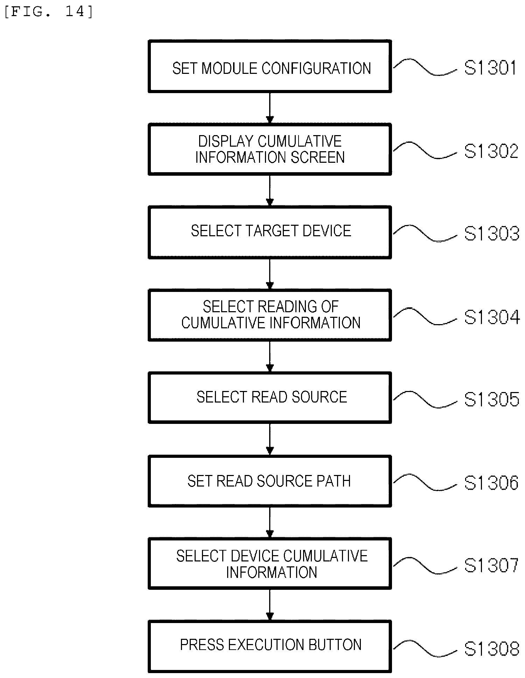

FIG. 14 is a flowchart of operating settings on the cumulative information setting screen 800 in step S1008 in FIG. 11.

First, the analysis unit 1012 is connected to the analysis unit 1013 in the automatic analysis system 100B of the system 2 (step S1301).

Next, a cumulative information button is selected on the system setting screen 700, and the cumulative information setting screen 800 is displayed (step S1302).

Then, whether to read cumulative information into a device of the analysis unit 1013 of the system 2 is selected in the device serial number selection field 801 (step S1303). Then, in order to read the cumulative information from the external medium 10 to the device, reading is selected in the information reading and writing selection field 802 (step S1304), and then the external medium 10 from which the cumulative information is to be read is selected in the information storage location selection field 803 (step S1305).

Then, in order to determine a path in detail of the external medium 10 specified for the cumulative information, a path is input to the information storage path input box 804 (step S1306). Cumulative information to be read from the external medium 10 is checked and selected from the respective cumulative information selection checkboxes 806 (step S1307). When the execute button 807 is pressed, since operations are completed on the cumulative information setting screen 800, a process of reading from the external medium 10 is started (step S1308).

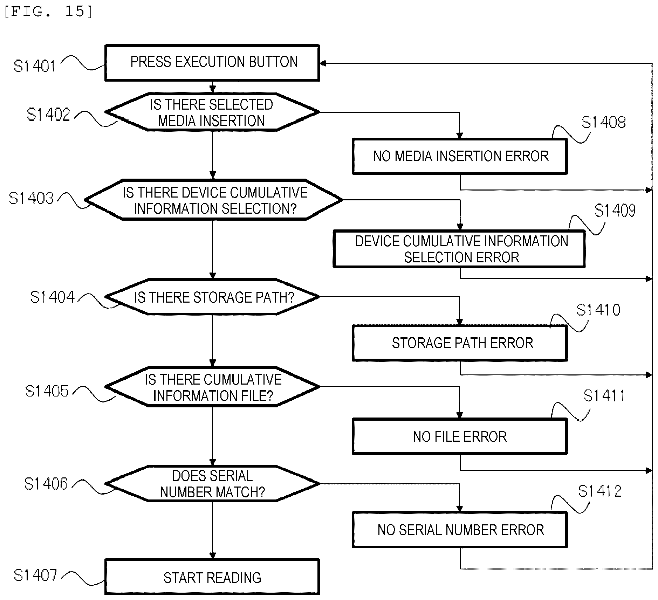

FIG. 15 shows a cumulative screen reading process, which is a processing flow after step S1308 in FIG. 14 is executed.

As shown in FIG. 15, when it is detected that the execute button 807 is selected on the cumulative information setting screen 800 (step S1401), in order to read cumulative information from the external medium 10 selected in the information storage location selection field 803, it is checked whether the external medium 10 is inserted into the output unit 305 (step S1402).

When the external medium 10 is not inserted, an error screen is displayed (step S1408), and the processing returns to step S1401. On the other hand, when the external medium 10 is inserted, the processing proceeds to step S1403.

Then, it is checked whether a writing target is checked in the respective cumulative information selection checkboxes 806 (step S1403). When none are checked, an error screen is displayed (step S1409), and the processing returns to step S1401. On the other hand, when the write target is checked, the processing proceeds to step S1404.

Next, when the cumulative information is written to the external medium 10, it is checked whether the path input in the information storage path input box 804 exists (step S1404). When the folder of the path does not exist, an error screen is displayed (step S1410), and the processing returns to step S1401. On the other hand, when the folder of the path exists, the processing proceeds to step S1405.

Next, it is checked whether a cumulative information file exists in the external medium 10 (step S1405). When no files exist, an error screen is displayed (step S1411), the processing returns to step S1401. On the other hand, when a file exists, the processing proceeds to step S1406.

Next, it is checked whether the serial number selected in the device serial number selection field 801 matches a serial number of the key information included in the cumulative information (step S1406). When the serial numbers do not match, an error screen is displayed (step S1412), and the processing returns to step S1401. On the other hand, when the serial numbers match, the processing proceeds to step S1407, and reading from the external medium 10 is started (step S1407).

Next, effects according to the present embodiment will be described.

The above described automatic analysis system 1 according to Embodiment 1 of the invention includes: two or more analyzers of a biochemical analyzer 110 and an immunoassay analyzer 210, which have unique identification information and cumulative information associated with the unique identification information, and analyze a sample; and a control device 300 that controls operations of the biochemical analyzers 110 and the immunoassay analyzer 210 and manages the unique identification information and the cumulative information of each of the biochemical analyzer 110 and the immunoassay analyzer 210. When the biochemical analyzer 110 and the immunoassay analyzer 210 in a system are rearranged, the control device 300 takes over the cumulative information that the newly introduced biochemical analyzer 110 and immunoassay analyzer 210 have in a pre-rearrangement system based on the unique identification information, and manages cumulative information in a new system based on the taken-over cumulative information.

Accordingly, it is possible to take over cumulative information even an analyzer is relocated to a different system, and to continuously manage operating time, maintenance, adjustment values, and the like of respective operation mechanisms from a pre-rearrangement analysis system even in a newly configured analysis system. Therefore, since a device status can be accurately and reliably understood, repair and maintenance can be accurately executed at an appropriate timing. Accordingly, it is possible to respond more appropriately to new demands such as reuse of a part of analyzers in a case of reselling an automatic analysis system when new demand arises, or in a case of changing a layout of a device configuration.

The automatic analysis system 1 and the information takeover method in the automatic analysis system according to the present embodiment are very suitable for dealing with systems currently in operation through software updates.

Further, since the automatic analysis system 1 further includes the display unit 303 that displays information of the automatic analysis system 1, and the control device 300 causes the display unit 303 to display the device configuration screens 600 and 650 for setting the unique identification information, the unique identification information can be easily set and checked.

Further, since the control device 300 causes the display unit 303 to display the cumulative information setting screen 800 on which cumulative information can be read and written, work of an operator when a takeover operation is performed can be facilitated, and takeover can be more accurately and reliably performed.

Further, since the control device 300 can write and read the unique identification information and the cumulative information to and from the external medium 10, unique identification information and cumulative information associated with the unique identification information can be easily backed up as appropriate, and work can be easily performed in a case of takeover.

Further, since the automatic analysis system 1 further includes the storage unit 302, and the control device 300 records a device serial number as unique identification information in the storage unit 302, serial numbers provided in a system can be basically reused, and can be easily applied to existing systems and devices.

Further, since the automatic analysis system 1 further includes the barcode reader 131 and 152, and the control device 300 can write out the unique identification information as a barcode, and manages the bar-coded unique identification information read by the barcode readers 131 and 152, a possibility of an input error can be eliminated and a possibility of an error occurring in a takeover operation can be reduced compared to a case of manual input by a service person or the like.

Further, since the external medium 10 is one or more of a USB medium, a CD medium, and a DVD medium, appropriate backup can be further performed, and a takeover operation can be facilitated.

### <Embodiment 2>

An automatic analysis system and an information takeover method in the automatic analysis system according to Embodiment 2 of the invention will be described below.

Unlike Embodiment 1 in which the automatic analysis system and the information takeover method in the automatic analysis system are suitably applied to systems currently in operation by software updates, the automatic analysis system and the information takeover method in the automatic analysis system according to the present embodiment are suitable for devices to be manufactured and shipped in the future.

When an analyzer in a system is to be rearranged, in order to more accurately take over cumulative information in the pre-rearrangement system, it is desirable to use unique identification information that does not overlap with that of other automatic analysis systems and analyzers.

Therefore, the device configuration screen 650 shown in FIG. 8 includes input fields for the ISE device serial number 657, the sampler device serial number 658, the biochemical device serial number 659, and the immune device serial number 660, and serial numbers unique to respective devices can be input, and in a system to be manufactured and shipped from now on, when serial numbers are assigned at time of shipment, the serial numbers are managed such that the serial numbers do not overlap with that of other systems and devices. The assigned device serial number is used as unique identification information to be associated with cumulative information.

Other configurations and operations are substantially the same as those of the automatic analysis system and the information takeover method in the automatic analysis system according to Embodiment 1 described above, and details thereof are omitted.

The automatic analysis system and the information takeover method in the automatic analysis system according to Embodiment 2 of the invention substantially have the same effects as in the automatic analysis system and the information takeover method in the automatic analysis system according to Embodiment 1 described above.

### <Embodiment 3>

An automatic analysis system and an information takeover method in the automatic analysis system according to Embodiment 3 of the invention will be described below.

In Embodiment 1, a case in which the external medium 10 is illustrated as a location that can be selected in the information storage location selection field 803 on the cumulative information setting screen 800, but in the present embodiment, the control device 300 is configured to be able to transmit cumulative information to a record medium of a host computer connected to the automatic analysis system 1 via a network instead of the external medium 10, and manages the cumulative information in the record medium of the host computer.

More specifically, the host system can be selected in the information storage location selection field 803 on the cumulative information setting screen 800. When the execute button 807 is selected, unique identification information input on the cumulative information setting screen 800 and cumulative information are transmitted to the host computer and managed by the host system.

Then, when an operation for requesting reading is performed, the control device 300 requests a check from the record medium of the host computer, and when cumulative information associated with unique identification information exists in the host system, the control device 300 receives the corresponding cumulative information.

Other configurations and operations are substantially the same as those of the automatic analysis system and the information takeover method in the automatic analysis system according to Embodiment 1 described above, and details thereof are omitted.

The automatic analysis system and the information takeover method in the automatic analysis system according to Embodiment 3 of the invention substantially have the same effects as in the automatic analysis system and the information takeover method in the automatic analysis system according to Embodiment 1 described above.

Further, since the control device 300 is configured to be able to transmit the cumulative information to the record medium of the host computer connected to the automatic analysis system 1 via the network, and manages the cumulative information in the record medium of the host computer, there is no room for a risk of loss of cumulative information due to loss that may occur in the external medium 10 or the like, and takeover can be performed more reliably.

Further, the control device 300 transmits the unique identification information input on the cumulative information setting screen 800 to the host computer. When the cumulative information associated with the unique identification information exists in the record medium of the host computer, the control device 300 receives the corresponding cumulative information, and the takeover can be reliably achieved.

### <Others>

The invention is not limited to the embodiments described above, and includes various modifications. The above embodiments are described in detail for easy understanding of the invention, and the invention is not necessarily limited to those including all the configurations described above.

Further, a part of a configuration of an embodiment may be replaced with a configuration of another embodiment, and a configuration of an embodiment may also be added with a configuration of another embodiment. Further, a part of a configuration of each embodiment may be added, deleted, or replaced with another configuration.

### Reference Signs List

1, 1A, 100A, 100B: automatic analysis system
10: external medium (external record medium)
101: sampler device
102: sample rack
102a: emergency sample rack
103: sample rack supply unit
104: sample rack accommodating unit
105: transport line
106: sample identification device
107: rack rotor
107a, 107b: slot
108: emergency sample rack loading unit
109: emergency sample rack waiting area
110: biochemical analyzer (analyzer)
111, 211: sample dispensing mechanism
112, 212: dispensing line
113, 213: sample identification device
114, 214: reagent disk
115, 215: reagent dispensing mechanism
116, 216: reaction disk
121, 221: top cover
122, 222: opening and closing detector
131, 152: barcode reader (barcode reader)
150: sampler device
151: rack accommodating unit
210: immunoassay analyzer (analyzer)
300, 300A, 300B: control device
301: controller
302: storage unit (internal record medium)
303: display unit
304: input unit
305: output unit
400: entire screen of automatic analysis system
401: status region
402: menu button
403A: global region
403B: local region
404: menu screen
405: system button
406: setup button
407: close button
500: setup screen
501: item allocation button
502: device power button
503: device configuration button
504: close button
600: device configuration screen (setting screen)
601: system name field
602: system serial number field
603: immune device connection radio button
604: immune device name
605: immune device abbreviation
606: immune device serial number
607: biochemical device connection radio button
608: biochemical device name
609: biochemical device abbreviation
610: biochemical device serial number
611, 661: registration button
612, 662: cancel button
650: device configuration screen (setting screen)
651: system name field
652: system serial number field
653: ISE device information region
654: sampler device information region
655: biochemical device information region
656: immune device information region
657: ISE device serial number
658: sampler device serial number
659: biochemical device serial number
660: immune device serial number
700: system setting screen
701: system setting button region
702: screen display button
703: service setting button region
704: cumulative information button
705: screen display button
706: close button
800: cumulative information setting screen (read-write screen)
801: device serial number selection field
802: information reading and writing selection field
803: information storage location selection field
804: information storage path input box
805: device cumulative information region
806: cumulative information selection checkbox
807: execute button
808: cancel button
1011: analysis unit of system 1
1012: analysis unit to be relocated
1013: analysis unit of system 2

## Claims

1. An automatic analysis system, comprising:
two or more analyzers having unique identification information and cumulative information associated with the unique identification information, and configured to analyze a sample; and
a control device configured to control operations of the analyzers and manage the unique identification information and the cumulative information of each of the analyzers,
wherein when the analyzers in a system are rearranged, the control device takes over the cumulative information that a newly introduced analyzer has in a pre-rearrangement system based on the unique identification information, and manages cumulative information in a new system based on the taken-over cumulative information.

2. The automatic analysis system according to claim 1, further comprising:
a display unit configured to display information of the automatic analysis system,
wherein the control device causes the display unit to display a setting screen for setting the unique identification information.

3. The automatic analysis system according to claim 2,
wherein the control device causes the display unit to display a read-write screen on which the cumulative information is readable and writable.

4. The automatic analysis system according to claim 1,
wherein the control device is able to write and read the unique identification information and the cumulative information to an external storage medium.

5. The automatic analysis system according to claim 1, further comprising:
an internal record medium,
wherein the control device records a device serial number on the internal record medium as the unique identification information.

6. The automatic analysis system according to claim 1, further comprising:
a barcode reader,
wherein the control device is configured to,
write out the unique identification information as a barcode, and
manage the bar-coded unique identification information read by the barcode reader.

7. The automatic analysis system according to claim 4,
wherein the external storage medium is one or more of a USB media, a CD media, and a DVD media.

8. The automatic analysis system according to claim 3,
wherein the control device is configured to,
transmit the cumulative information to a record medium of a host computer connected to the automatic analysis system via a network, and
manage the cumulative information on the record medium of the host computer.

9. The automatic analysis system according to claim 8,
wherein the control device is configured to,
transmit the unique identification information input on the read-write screen to the host computer, and
when the record medium of the host computer includes the cumulative information associated with the unique identification information, receive the relevant cumulative information.

10. An information takeover method in automatic analysis system, comprising:
when two or more analyzers configured to analyze a sample are rearranged, taking over cumulative information associated with unique identification information that a newly introduced analyzer has in a pre-rearrangement system based on the unique identification information in the system, and managing cumulative information in the analyzers in a new system based on the taken-over cumulative information.
